# EUROPEAN PATENT SPECIFICATION

(11) **EP 3 551 198 B1**
(45) Date of publication and mention of the grant of the patent: **02.02.2022**
(21) Application number: 16923314.5
(22) Date of filing: 08.12.2016
(51) Int. Cl.: A61K 9/08, A61K 47/36, A61K 47/34

(54) **COMPOSITIONS AND METHODS FOR TREATMENT OF LOSS OF FLUIDS LEADING TO HYPOTENSION AND/OR HYPOVOLEMIA**
ZUSAMMENSETZUNG UND VERFAHREN ZUR BEHANDLUNG DES VERLUSTES VON FLÜSSIGKEITEN MIT FOLGENDER HYPOTONIE UND/ODER HYPOVOLÄMIE
COMPOSITION ET MÉTHODES POUR LE TRAITEMENT DE PERTE DE FLUIDES CONDUISANT À L'HYPOTENSION ET/OU L'HYPOVOLÉMIE

(43) Date of publication of application: 16.10.2019
(73) Proprietor: Cellphire Inc., Rockville, MD 20850 (US)
(72) Inventor: FEUERSTEIN, Giora, Bryn Mawr, PA 19010 (US); WILLARD, Stephen, H., Chevy Chase, MD 20815 (US); JORDA, Rafael, H., 33700 Merignac (FR)
(74) Representative: Fish & Richardson P.C.
(86) International application number: PCT/US2016/065681
(87) International publication number: WO 2018/106250

(56) References cited:
- WO-A1-2016/201081
- WO-A2-2006/020773
- US-A- 5 571 801
- US-A- 5 723 281
- US-A1- 2001 028 880
- US-A1- 2006 034 809
- US-A1- 2007 243 178
- US-A1- 2013 059 380
- US-A1- 2016 235 781

## Description

### BACKGROUND OF THE INVENTION

### Field of the Invention

The present invention relates to the field of medicine. More specifically, the present invention relates to compositions and methods for treatment of subjects suffering from loss of fluids that causes reduced volume and/or pressure in the systemic circulation, and for treatment of organ conditions where blood flow is impaired or insufficient, resulting in organ distress, including tissue loss, morbidity, and mortality.

### Description of Related Art

Solutions containing high molecular weight polymers, such as Hextend^{®} (Hospira, Lake Forest, IL), which is a 6% hetastarch (hydroxyethyl starch; HES) in lactate electrolyte injection formulation, are known in the art. Hextend^{®} is approved by the U.S. Food and Drug Administration (FDA) for clinical use for the purpose of treatment of hypovolemia when plasma volume expansion is desired. Hextend^{®} is used in military venues as well as in civilian health management facilities. However, on 25 November 2013, the U.S. FDA announced that, due to increased mortality and/or severe renal injury in certain patient populations treated with HES, a "Boxed Warning" must be placed on products containing HES. In addition, it has been documented that HES solutions should be protected from freezing and should not be exposed to temperatures above 40°C for extended periods of time.

Human albumin solutions (USP, 5% or 25%), such as those sold under the brand name Albutein^{®} (Grifols Biologicals, Inc., Los Angeles, CA) are also known for their use in treating hypovolemic shock and as an adjunct in hemodialysis for patients undergoing repeated long-term dialysis or for those patients who are fluid-overloaded and cannot tolerate substantial volumes of salt solution for therapy of hypovolemia, shock, or hypotension. However, human albumin solutions are not widely used due to the cost, logistic limitations, and because such solutions cannot be frozen or exposed to a temperature over 30°C.

Hartmann's solution, also known as compound sodium lactate (CSL), Ringer's lactate, 0.9% saline, and 5% dextrose/normal saline are electrolyte-based solutions known in the art for blood volume replacement. In addition, two hypertonic saline solutions (3%, 5%) have been used for volume replacement, but it is now recognized that such solutions have the drawback of delivering high sodium and chloride ions loads systemically, which could result in adverse effects due to systemic sodium chloride overload.

Hypertonic saline (HTS) 7.5% is another treatment for blood volume expansion by introducing extracellular volume into intravascular space similarly to Hextend^{®} and HTS 3% and HTS 5%. HTS 7.5% has the same medical utility as these other compositions, but requires only one-eighth the volume of saline or lactated Ringer's solution, thus resulting in less carrying weight for medics in a war theater and less storage requirements for emergency personnel, *e.g.*, first responders, such as EMTs. Using HTS 7.5%, two infusions of 250 ml can be used to treat hypovolemia cases. While HTS 7.5% has been approved and tested, it is of limited use. Substitutes, such as HTS 3% and HTS 5%, can be used instead and can be stockpiled long term. As is the case with Hextend^{®}, one of the weaknesses of HTS is the questionable effectiveness when extracellular volumes are low in injured patients or in strenuous conditions, such as dehydration. Also, the high sodium chloride loads resulting from HTS 7.5% use could carry risks in certain medical states, such as heart or renal failure conditions.

### SUMMARY OF THE INVENTION

In general, the present invention is directed to a composition that is useful in medical treatments for subjects who are suffering, or are suspected will soon suffer, from loss of fluids that causes reduced volume and/or pressure in the systemic circulation. The reduced volume and/or pressure can, but is not necessarily, accompanied by life threatening lower than normal blood pressure. In addition, the present invention includes a composition that is useful in other medical treatments, such as treatment of organ conditions where blood flow is impaired or insufficient, resulting in organ distress, including tissue loss, organ loss, morbidity, or even death. In general, the composition of the invention comprises salt, preferable inorganic salt, and a high molecular weight, non-ionic, hydrophilic polymer. In embodiments, the composition can contain additional substances including, but not limited to, a natural or synthetic sugar and a buffering substance. The composition can be a solid composition or a liquid composition. The composition, when in liquid form, can be hypertonic, isotonic, or hypotonic in regards to its osmotic pressure as compared to the subject's intravascular (blood/plasma) and extravascular (extracellular) fluids. Preferably, the composition is isotonic. The composition has the capability to increase intravascular volume and pressure of the treated subject. The composition has resuscitative effects, and is thus particularly well suited for use in states of low blood volume and pressure. When manufactured as a solid, the composition can be in the form of a dry powder or lyophilized "cake". While the dry composition can be produced using any suitable method of mixing the components, including simply by thoroughly mixing the components in their solid state using mechanical means, in exemplary embodiments disclosed herein, the dry composition is prepared first as a homogeneous liquid composition, then freeze-dried (lyophilized). In other embodiments, the liquid composition is dried using vacuum and/or heat evaporation, such as vacuum drying, spray drying, or spray freeze drying. In use, the composition is typically an aqueous composition for infusion or injection into the subject. However, it is to be understood that the composition, as prepared, is often a solid composition that is suitable for hydration, solubilization, etc. with a liquid, such as an aqueous liquid.

The invention also is directed to a method of therapeutic restoration of blood volume and pressure states, resuscitation of low blood volume and pressure states, or both. In one aspect, the present invention provides a method for treating loss of fluids in a subject that causes reduced volume and/or pressure in the systemic circulation. In general, the method comprises administering to the subject an amount of the composition of the invention that is effective to restore, at least to a measurable extent, the subject's blood volume and/or pressure state, and in embodiments, the subject's body fluid balance.

The invention further is directed to a method of prophylactic treatment of a subject to protect against conditions that can cause systemic or specific organ failure, which can result in death of the subject. According to this aspect of the invention, the method comprises administering to the subject an amount of the composition of the invention that is effective to protect against future systemic or specific organ failure or the death of the subject. As with the therapeutic method, the prophylactic method provides increased blood volume over the subject's blood volume prior to administration of the composition of the invention, and the increased blood volume prevents or reduces the chance of impaired organ function or organ failure in cases where organ failure or death due to organ failure can be expected. In embodiments, the prophylactic method is practiced on subjects who are to be subjected to radiocontrast agents for imaging tissues or organs, such as arteries, where the radiocontrast agents are known to be associated with Acute Kidney Injury (AKI). In other embodiments, the prophylactic method is used to expand blood volume in subjects about to undergo surgery.

In another aspect, the invention is directed to a composition and therapeutic method of treating traumatic brain injury (TBI). In general, the method comprises administering to a subject suffering from TBI an amount of the composition of the invention that is effective to reduce neuronal cell death as compared to the amount of neuronal cell death that would result in the absence of administration. In preferred embodiments, the amount of the composition that is administered is also an amount that is sufficient to restore, at least to a measurable extent, the subject's blood volume and/or pressure state, if needed. In a preferred embodiment, the amount of the composition that is administered is also an amount that is sufficient to secure, at least to a measurable extent, the subject's physiological range (or mitigate the risk of an excessive increase in the intra-cranial pressure) of intra-cranial pressure, which can mitigate the risks of unwanted consequences of cerebral edema, a complication of TBI that could result in death.

As will be apparent to the practitioner, the invention also encompasses use of compositions of the invention for therapeutic or prophylactic treatment of a subject suffering from, or expected to suffer from, loss of fluids in the subject that causes reduced volume and/or pressure in the systemic circulation. The reduced volume and/or pressure in the systemic circulation can be, but is not necessarily, associated with lower than normal blood pressure. The treatment can also or alternatively be a treatment of organ conditions where blood flow is impaired or insufficient, resulting in organ distress (e.g., insufficient blood flow to the heart, brain, or kidneys), including tissue loss, morbidity, and mortality. The present invention thus encompasses compositions for use as a medicament and compositions for use in the treatment of the effects of reduction of or loss of a subject's blood volume and/or pressure state.

As discussed above, broadly speaking, the invention encompasses a composition comprising at least one salt and at least one high molecular weight, non-ionic, hydrophilic polymer. For ease of future reference, this aspect of the invention is referred to as the "basic composition". It surprisingly has been found that a combination of at least one salt and at least one high molecular weight, non-ionic, hydrophilic polymer is sufficient to provide resuscitative effects to treat loss of blood volume, pressure state, and to help ameliorate the effects of edema in TBI cases. It was further surprising that these ameliorating effects are not associated with adverse events, as have been seen with other colloidal compositions. Therefore, because the biological effects are substantially or completely due to these two substances, in embodiments, the basic composition consists essentially of at least one salt and at least one high molecular weight, non-ionic, hydrophilic polymer. In embodiments of the invention, the composition comprises, in addition to the at least one salt and at least one high molecular weight, non-ionic, hydrophilic polymer, at least one saccharide, at least one buffer, and/or at least one other biologically tolerable substance. Included in these additional components are bioactive agents, such as drugs and nutrients. Preferably, the additional components do not comprise blood cells or substances derived from blood cells. For ease of future reference, these embodiments of the invention are referred to herein as "complex compositions". In embodiments, the at least one saccharide comprises trehalose. In embodiments, the at least one high molecular weight, non-ionic, hydrophilic polymer is a co-polymer of sucrose and epichlorohydrin. In exemplary embodiments, the complex composition comprises HEPES buffer, NaCl, KCl, NaHCO₃, dextrose, trehalose, and a co-polymer of sucrose and epichlorohydrin. In some embodiments, the composition is in a solid state, such as a dry powder, while in other embodiments, the composition is in a liquid state. In general, in both the basic composition and the complex compositions, the high molecular weight, non-ionic, hydrophilic polymer is present in an amount from approximately two percent (w/v) to approximately six percent (w/v) or even up to approximately 10 percent (w/v) when in a liquid state.

In embodiments of the basic composition, the composition comprises normal saline or another isotonic crystalline composition and at least one high molecular weight non-ionic hydrophilic polymer, which is present in an amount from approximately two percent (w/v) to approximately six percent (w/v) or even up to approximately 10 percent (w/v) when in a liquid state.

The compositions, whether in a solid state or a liquid state, can be combined with one or more elements, such as medical equipment or supplies useful for injecting or infusing the composition into a subject to form a kit. In embodiments, the kit is a "ready to use" kit that includes all of the components of a liquid composition according to the invention along with all of the equipment required to administer the composition to a subject in need. The kit can also comprise, as two separately packaged elements, a solid composition of the invention in one container and a suitable liquid (e.g., sterile water for injection) in a second container. In use, the composition and the liquid are combined to make a liquid composition and administered to the subject, such as by injecting or infusing the liquid composition into the subject.

The invention thus further encompasses a method for treating loss of fluids in a subject in need thereof, wherein the loss of fluids causes reduced volume and/or pressure in the subject's systemic circulation. In embodiments, the method comprises administering to the subject the basic or complex composition in liquid form in an amount that is effective to increase, at least to a measurable extent, the volume and/or pressure in the subject's systemic circulation. When a complex composition is administered, the composition does not comprise blood cells or substances derived from blood cells. In embodiments, administering the composition to the subject partially or completely restores blood flow to at least one organ in the subject. In embodiments, the blood volume and/or blood pressure and/or organ blood flow of the subject before administration of the composition are compromised or likely to become compromised as compared to normal levels. In embodiments, the step of administering comprises intravenous infusion or injection of the liquid composition into the subject. In some embodiments, the subject is suffering from a disease or disorder that does not have a clinical symptom relating to blood volume or blood pressure. The method of the invention is suitable for use in subjects that have at least one of the following non-limiting conditions: hypovolemia, excessive sodium ions, and excessive extracellular volume/fluid (ECV/F). In the case of hypovolemia, it can be caused by at least one of the following conditions: blood loss by hemorrhage following trauma, injury, or a medical condition (*e.g*., hemophilia) or intervention; dehydration due to heat, and fluid loss associated with a burn injury. The method of the invention is also applicable to subjects suffering from a disease caused by an infectious agent that causes loss of blood pressure or blood volume, such as one that causes Viral Hemorrhagic Fever (VHF), including an Ebola virus or other sepsis conditions. The compositions and methods of the invention have utility in treating both humans and non-human animals. In addition, the methods of the invention can be therapeutic or prophylactic methods of treatment of subjects in need of, or suspected of being in need of in the future, treatment. In exemplary embodiments, the method is practiced using an aqueous basic composition that consists essentially of at least one salt and at least one high molecular weight, non-ionic, hydrophilic polymer. In other exemplary embodiments, the method is practiced using a complex composition that comprises HEPES buffer, NaCl, KCl, NaHCO₃, dextrose, trehalose, and a co-polymer of sucrose and epichlorohydrin. Yet further, the complex composition can include, without limitation, CaCl₂ and/or MgCl₂.

The present invention addresses the long-felt need in the medical field by providing solid and liquid compositions that are safe, can have a shelf-life beyond three years, do not require refrigeration (also known in the art as "cold-chain" storage), are easily transported, have superior resuscitative properties as compared to certain resuscitative compositions currently commercially available, and are simple to use. The solid compositions of the invention can easily be hydrated at the time they are needed, such as for medical treatments discussed herein. The liquid compositions of the invention provide a solution for the widely recognized need in the art for a resuscitative fluid that can be stocked in large numbers as a solid composition and that can be hydrated with liquids (*e.g*., sterile water) that are routinely stocked in hospitals and other medical centers, including all levels of military echelons.

### BRIEF DESCRIPTION OF THE DRAWINGS

Fig. 1 is a line graph showing a rapid, robust, and persistent increase in blood pressure upon treatment with a complex composition according to the invention in a rat model of hemorrhagic hypotension, where the volume of the composition infused into the rat was equal to the amount of blood lost.
Fig. 2 is a line graph showing that a complex composition of the invention provides a sustained increase in blood pressure in a rat model of hypotension for at least two hours, and that the increase is significantly superior to the increase achieved using normal saline, where the volume of the complex composition and the volume of saline infused into the rat is equal to the amount of blood lost and the amount and rate of infusions of the two treatments are the same.
Fig. 3 is a line graph showing that a complex composition of the invention is effective at rapidly increasing blood pressure in a rat model of hypotension when administered to rats at a volume that is 50%, 33%, and 25% of the volume of blood lost.
Fig. 4 is a line graph showing that a complex composition of the invention provides a sustained increase in blood pressure in a rat model of hypotension for at least two hours, and that the increase at that time point is superior to the increase achieved using an equal volume of HAES-steril^{®} (6% hydroxyethyl starch; Fresenius KGaA, Germany).
Fig. 5 is a line graph showing that a complex composition of the invention having Ficoll400 in a concentration of 6% (w/v) and a complex composition of the invention having Ficoll400 in a concentration of 2% (w/v) are both effective at rapidly increasing blood pressure in a rat model of hemorrhagic hypotension when administered to rats in an amount equal to the amount of blood lost.
Fig. 6 is a line graph showing a comparison of a complex composition and a basic composition and showing that they are essentially equivalent in increasing blood pressure in a rat model of hypotension when administered to rats in an amount equal to the amount of blood lost.
Fig. 7 is a line graph showing a comparison of a complex composition and a basic composition and showing that they are essentially equivalent in increasing blood pressure in a rat model of hypotension when administered to rats in an amount that is 25% of the amount of blood lost.

### DETAILED DESCRIPTION OF VARIOUS EMBODIMENTS OF THE INVENTION

Reference will now be made in detail to various exemplary embodiments of the invention, data supporting the invention being illustrated in the accompanying figures. It is to be understood that the following discussion of exemplary embodiments is not intended as a limitation on the invention, as broadly disclosed herein. Rather, the following discussion is provided to give the practitioner a more detailed understanding of certain aspects and features of the invention.

Before embodiments of the present invention are described in detail, it is to be understood that the terminology used herein is for the purpose of describing particular embodiments only, and is not intended to be limiting. Further, where a range of values is provided, it is to be understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention.

Unless defined otherwise, all technical and scientific terms used herein have the same meaning as commonly understood by one of ordinary skill in the art to which the term belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited. The present disclosure is controlling to the extent it conflicts with any incorporated publication.

As used herein and in the appended claims, the singular forms "a", "an", and "the" include plural referents unless the context clearly dictates otherwise. Thus, for example, reference to "a high molecular weight, non-ionic, hydrophilic polymer" includes a plurality of such polymers and reference to "the basic composition" includes reference to one or more basic compositions and equivalents thereof known to those skilled in the art, and so forth. Furthermore, the use of terms that can be described using equivalent terms include the use of those equivalent terms. Thus, for example, the use of the term "subject" is to be understood to include the terms "animal", "human", "patient", and other terms used in the art to indicate one who is subject to a medical or veterinary treatment. As another example, the use of the term "composition" is to be understood to include the terms "formulation", "mixture", "blend", and other terms used in the art to indicate a combination of two or more substances, which can, but do not necessarily, include a solvent, such as water.

The present invention provides compositions and methods for treating loss of fluids, hypotension due to loss of fluids, or other conditions that cause reduced volume and/or pressure in the systemic circulation, as well as conditions where blood pressure is normal yet an increase in blood volume might be useful, such as in the case of a radiocontrast agent. Such a reduction can lead to poor organ perfusion in a subject, including poor organ perfusion that leads to morbidity or death. Compositions having a similar formulation as that of certain complex compositions of the present invention were originally formulated as part of an invention directed to a hemostatic product comprising platelets or platelet-derived substances (see, for example, U.S. Patent No. 8,486,617). Within the context of the prior invention, the complex compositions of the present invention had no known utility apart from providing a physiologically compatible environment suitable for loading or surrounding platelets with substances that protect them during freeze-drying (lyophilizing) and could be safely infused intravenously after reconstitution. The present invention discloses the unexpected, serendipitous discovery of a therapeutic and prophylactic property of the present compositions in restoration and resuscitation of low blood volume and pressure states or states where organ perfusion is low, and in prevention of low blood volume and pressure states or states where organ perfusion is predicted to be low. As such, the present invention addresses a different medical need (blood volume expansion and blood pressure increase) from the prior invention (hemostasis).

Stated another way, the present invention relates to the unexpected and surprising finding that a composition similar to that of a prior invention, but lacking what was recognized in that prior invention as the biologically active components (*i.e.,* lacking platelets, platelet microparticles, or other platelet-derived substances needed for hemostasis), can have advantageous properties independent of the previously disclosed hemostatic product. The invention also relates to the unexpected and surprising finding that the compositions of the present invention, unlike certain other compositions for treatment of hypovolemia and hypotension that contain high-molecular polymers having colloidal properties, are not associated with impairment of renal function or other deficiencies (see Table 1, below).

In one general aspect, the invention provides compositions. The compositions can be either basic compositions or embodiments of the basic compositions that are more complex in their chemical make-up. The compositions can exist in either a solid state or liquid state. In preferred embodiments, the compositions are stored as solids, then hydrated just prior to use. In exemplary embodiments discussed herein, the solid composition is a "dry" composition, such as one made using lyophilization. As used herein, a solid composition is considered to be "dry" if it contains 2% or less moisture. Dry compositions of the present invention have the advantage of a relatively long shelf life. In certain exemplary embodiments discussed herein, a liquid composition is an aqueous composition formed by way of re-hydrating a lyophilized solid composition of the invention. Alternatively, a liquid composition can be freshly prepared for use shortly after preparation, without the need to lyophilize then re-hydrate.

It is thus to be understood that the solid compositions are suitable for hydration to provide a liquid composition. In embodiments relating to liquid compositions, preferably, the compositions are aqueous and suitable for safe administration to living subjects. The compositions can provide therapeutic or prophylactic effects when administered to a subject suffering from, or expected to suffer from, loss of fluids, as mentioned above. The loss of fluids can result in lower than normal blood pressure, lower than normal blood volume, lower than normal organ perfusion, or any combination of these. The compositions find, among other uses, utility in treatment of organ conditions where blood flow is impaired or insufficient, resulting in organ distress, including tissue loss and impaired organ function, which could lead to morbidity or death. Because the composition of the invention can be used to alleviate dangerous medical conditions, the composition can be thought of as a life-saving medical remedy.

In some preferred embodiments, the compositions are prepared, sterilized, and stored as liquid compositions. Because the liquid compositions have no biological components and are sterile, they have a relatively long shelf-life as compared to other liquid resuscitative compositions known in the art.

As discussed above, a basic composition according to the invention comprises or consists essentially of at least one salt and at least one high molecular weight, non-ionic, hydrophilic polymer. Additionally, as discussed above, a complex composition according to certain embodiments of the invention comprises at least one salt, at least one high molecular weight, non-ionic, hydrophilic polymer, and at least one saccharide, and optionally can include at least one buffering component. As such, a composition according to the invention can comprise one salt, two different salts, three different salts, etc. Likewise, a composition according to the invention can comprise one high molecular weight, non-ionic, hydrophilic polymer, two different high molecular weight, non-ionic, hydrophilic polymers, three different high molecular weight, non-ionic, hydrophilic polymers, etc. Further, a complex composition can comprise one saccharide, two different saccharides, three different saccharides, etc. It is to be understood that dry compositions according to the present invention are formulated with the prior knowledge of the volume of liquid composition to be created from the dry composition. As such, the amounts of each component of the composition can be calculated based on the molecular weights of the compounds and the pre-selected volumes. For example, a given dose of a solid composition will be hydrated with the appropriate amount of liquid. It is to be understood that in some embodiments, compositions of the invention, whether in a solid state or dry state or hydrated state, do not include platelets, platelet-derived material, or other components of blood.

While any salts can be used at concentrations that are biologically tolerable for a subject to be treated, in preferred embodiments, the salt is a sodium salt, a potassium salt, a phosphate salt, a magnesium salt, a calcium salt, or any other salt known to be present in blood (such as "trace elements"), or combinations of these exemplary salts. As with other components of the composition, the identity of the salt(s) in the composition is not critical as long as the salt(s) are present in amounts that are not toxic to the subject when the composition is administered to the subject. In some embodiments, the total salt concentration approximates or is identical to the salt isotonicity of the blood of the subject into which it will be administered. In some embodiments, however, the total salt concentration is hypertonic or hypotonic to the salt isotonicity of the blood of the subject into which it will be administered. Salts are each present in the liquid compositions of the invention at concentrations ranging from about 1 mM to about 250 mM. For example, a salt can be present at a concentration of 4 mM, 5 mM, 10 mM, 20 mM, 50 mM, 75 mM, 100 mM, 125 mM, or 150 mM, or about any of those concentrations. The skilled artisan will understand that all other specific values and ranges for concentrations within the disclosed ranges and specific values are inherently disclosed by these values and the ranges they represent without the need to disclose each specific value or range herein.

The saccharide(s) present in the complex composition can be any suitable saccharide, including a monosaccharide, disaccharide, or low molecular weight polysaccharide (*i.e.,* a polysaccharide having a molecular weight of less than about 20,000). In embodiments, two or more different saccharides are present in the complex composition. Non-limiting examples of suitable saccharides are sucrose, maltose, trehalose, glucose/dextrose, mannose, lactose, and xylose. In exemplary embodiments, the saccharide is the di-saccharide trehalose. The saccharide may be present in the complex composition in any suitable amount. For example, it may be present in liquid compositions in an amount of 1 mM to 1 M. In embodiments, each saccharide is present in a complex liquid composition in an amount of from 5 mM to 500 mM, such as from 10 mM to 500 mM. In some embodiments, each is present in an amount of from 20 mM to 200 mM. In embodiments, each is present in an amount from 40 mM to 100 mM, while in other embodiments, the saccharide is present in an amount from 50 mM to 150 mM. In certain particular embodiments, each saccharide is present in the complex composition in an amount of at least or about any of the following concentrations: 5 mM, 10 mM, 25 mM, 40 mM, 50 mM, 60 mM, 70 mM, 80 mM, 90 mM, and 100 mM. Of course, in various embodiments, the saccharide is present in different specific concentrations within the ranges recited above, and one of skill in the art can readily understand the various concentrations encompassed by the ranges and specific concentrations within those ranges without the need to specifically recite each herein. Likewise, for complex solid compositions, the amount of saccharide is adjusted depending on the desired final volume of the complex composition when an aqueous solvent is added. In situations where more than one saccharide is present in the complex composition, each saccharide may be present in an amount according to the ranges and particular concentrations recited above, or the total saccharide content can be according to the ranges and particular concentrations recited above.

Various high molecular weight, non-ionic, hydrophilic polymers are known in the art, and any such polymer(s) may be used in accordance with the present invention. In general, a high molecular weight polymer according to the present invention has a molecular weight (or an average molecular weight) of at least 70,000, such as at least or about 75,000, 80,000, 90,000, 100,000, 120,000, 130,000, 150,000, 200,000, 250,000, 300,000, 350,000, 400,000, 450,000, 500,000, 600,000, 1,000,000, 5,000,000, 10,000,000, or higher. As the skilled artisan will recognize, such high molecular weight polymers typically have a dispersion of molecular weights about a mean value. It is thus to be understood, for example, that reference herein to a polymer having a molecular weight of 400,000 indicates a collection of polymers having an average molecular weight of 400,000.

Non-limiting examples of high molecular weight, non-ionic, hydrophilic polymers according to certain embodiments of the invention include: co-polymers of sucrose and its derivatives, such as a synthetic polymer of sucrose and epichlorohydrin (available under the trade names of Polysucrose 400, Ficoll 400, Ficoll PM400, Ficoll 70, Ficoll PM70, and Ficoll-Paque (including "Plus" and "Premium")), poly(sucrose 1-O-methacrylate), poly(N-(methacrylamido)-1',2,3,3',4,4',6-hepta-O-benzyl-6'-amino-6'-deoxysucrose), and poly(6,6'-dideoxy-6,6'-N-dimethacryloyloxy ethylureido sucrose)-co-polystyrene; co-polymers of epichlorohydrin, such as polyepichlorohydrin, poly[(bisphenol A)-co-epichlorohydrin], and polystyrene-block-polyepichlorohydrin; polyethylene glycols, such as polyethylene glycol (PEG), poly(ethylene glycol) diacrylate, monofunctional PEGs, such as poly(ethylene glycol) bis(2-bromoisobutyrate), and PEG co-polymers, such as poly(styrene)-block-poly(ethylene glycol). Other non-limiting examples according to certain embodiments include polyethylene oxide(s), polyacrylamide(s), polyvinyl alcohol(s), polyvinylpyrrolidone(s), and poloxamers(s); and cellulose(s), including modified celluloses, such as cellulose acetate, 2-hydroxyl cellulose, ethyl cellulose, hydroxymethyl cellulose, hydroxyethyl cellulose, hydroxypropyl cellulose, hydroxypropyl-ethyl cellulose, hydroxypropyl-methyl cellulose, and sodium carboxymethyl cellulose. Yet further non-limiting examples of high molecular weight, non-ionic, hydrophilic polymers according to embodiments of the invention include: starches, such as starch, crosslinked starch, amylopectin, amylose, and modified starches, such as maltodextrin dextrose; dextrans, such as Class 1, Class 2 (Alternans), and Class 3 (Mutans) dextrans, and modified dextrans, such as phenyl-dextran; gelatins; and gums, such as gum Arabic.

The high molecular weight, non-ionic, hydrophilic polymers can thus be polysugars or co-polymers of sugars; starches and derivatives of starches; and pegylated derivatives of high molecular weight, non-ionic, hydrophilic polymers. The polymers can be carbohydrates or polymers that contain a carbohydrate portion. The polymers can further be branched or unbranched. The polymers can be hydrogels. In embodiments, they are biodegradable, allowing for immediate action when administered to a subject, but removal from the body via renal excretion or liver metabolism as natural blood production restores blood volume and blood pressure.

High molecular weight polymers having a molecular weight of about 70,000 or below, such as albumin and dextran 40/70, are known in the art for use in treatment of hypovolemia due to their colloidal properties. However, albumin is very expensive to make, requires sterility measures associated with blood products, and has a relatively short half-life in liquid form. Dextran 40 and 70 have been reported to be associated with anaphylactic reactions and hematologic adverse effects, such as prevention of platelet aggregation and facilitation of fibrinolysis, two properties that are undesirable when treating a bleeding subject. In recognition of these issues, Dextran 1 was introduced in order to test the sensitivity of individual to Dextran products prior to their use. The present invention excludes such polymers.

While not being held to any particular mode of action for the compositions of the invention, it is possible that the viscosity of the compositions might affect the resuscitative property of the compositions. To investigate this characteristic, a complex composition was manufactured and the dynamic viscosity was measured using a capillary viscometer (cannon-fenske 475A) in a water bath heated to 40°C ± 1°C. The dynamic viscosity under these conditions was determined to be 1.55 cP.

As an example of a safety profile of a composition of the present invention, Table 1 presents the results of a study performed in canines to determine the effect of a complex composition on renal function two days after infusion and 15 days after infusion of 13 ml/kg into the canines. As a measure of kidney safety, plasma creatinine was assayed at two days and at 15 days after exposure of canines to the composition. As Table 1 demonstrates, no change in plasma creatinine was noted, indicating no deterioration of renal function.

**Table 1: Summary Of Creatinine Levels In Canines**

| Time Point | Mean Creatinine Level | Standard Deviation | Number of Canines |
|---|---|---|---|
| Pre-Infusion | 0.51 | 0.052 | 12 |
| 2 Day Post-Infusion | 0.56 | 0.029 | 6 |
| 15 Day Post-Infusion | 0.58 | 0.079 | 6 |

The high molecular weight, non-ionic, hydrophilic polymer is included in liquid compositions at an amount of from 1% to 30% (w/v), such as from 5% to 30%, 5% to 20%, and 5% to 10%. In embodiments, the high molecular weight, non-ionic, hydrophilic polymer is present in the composition at a final concentration of 1%, 2%, 3%, 4%, 5%, 6%, 7%, 8%, 9%, or 10% (w/v). In certain embodiments, the high molecular weight, non-ionic, hydrophilic polymer is present in the composition in a final concentration of 4% - 8% (w/v).

In some embodiments, a buffering component is included in a complex composition or in a basic composition consisting essentially of at least one salt and at least one high molecular weight, non-ionic, hydrophilic polymer. The buffering component may be any buffer that is biologically tolerable by the subject into which it will be administered at the concentration and volume used, as well as its frequency over time. Thus, the buffer may comprise any of the known biologically compatible buffers available commercially, such as phosphate-buffered saline (PBS) and Tris-based buffers, such as Tris-buffered saline (TBS). Likewise, the composition may comprise one or more of the following buffers: propane-1,2,3-tricarboxylic (tricarballylic); benzenepentacarboxylic; maleic; 2,2-dimethylsuccinic; EDTA; 3,3-dimethylglutaric; bis(2-hydroxyethyl)imino-tris(hydroxymethyl)-methane (BIS-TRIS); benzenehexacarboxylic (mellitic); N-(2-acetamido)imino-diacetic acid (ADA); butane-1,2,3,4-tetracarboxylic; pyrophosphoric; 1,1-cyclopentanediacetic (3,3 tetramethylene-glutaric acid); piperazine-N,N'-bis-(ethanesulfonic acid) (PIPES); N-(2-acetamido)-2-amnoethanesulfonic acid (ACES); 1,1-cyclohexanediacetic; 3,6-endomethylene-1,2,3,6-tetrahydrophthalic acid (EMTA; ENDCA); imidazole; 2-(aminoethyl)trimethylammonium chloride (CHOLAMINE); N,N-bis(2-hydroxyethyl)-2-aminoethanesulfonic acid (BES); 2-methylpropane-1,2,3-triscarboxylic (beta-methyltricarballylic); 2-(N-morpholino)propane-sulfonic acid (MOPS); phosphoric; N-tris(hydroxymethyl)methyl-2-amminoethane sulfonic acid (TES); N-2-hydroxyethylpiperazine-N'-2-ethanesulfonic acid (HEPES), and bicarbonate (*e.g*., NaHCO₃). Furthermore, the buffer component can provide buffering capacity in the range of pH 4 to pH 10 for liquid compositions.

In embodiments, a composition according to the invention can comprise about 7.0 - 9.5 (such as 7.0 - 7.5) mM HEPES, about 50 - 70 (such as 50 - 60) mM NaCl, about 3.5 - 4.5 (such as 3.5 - 4.0) mM KCl, about 8 - 10 (such as 9 - 9.5) mM NaHCO₃, about 50 - 150 (such as about 75 - 100) mM trehalose, about 3 - 5 (such as 3.5 - 4.5) mM dextrose, and about 5.0 -7.0 (such as about 5.5 - 6.5) w/v polysucrose 400. When a buffer is used, the pH of the composition is typically about 6.5 - 7.0 (such as about 6.8).

In embodiments, the complex composition of the invention is a solid composition and has a single glass transition temperature (Tg), as determined using differential scanning calorimetry, at about 90°C, such as at 90.5°C, whereas in other embodiments the T_{g} is about 96°C, such as 95.6°C. In embodiments, complex solid compositions show a single T_{g} at around 90°C to 100°C. In other embodiments, the complex composition of the invention is an aqueous liquid composition and has a single T_{g} at about -30°C, such as -30.11°C, as determined using differential scanning calorimetry.

In another aspect, the invention provides a method for preparing a basic composition of the invention. In general, the method comprises mixing together, in any order, at least one salt and at least one high molecular weight, non-ionic, hydrophilic polymer to create a uniformly dispersed composition. Where desired, one or more substances that provide buffering capacity can also be added as part of the composition. In embodiments, the method can include preparing a mixture in an aqueous solvent. In these embodiments, the method comprises adding the at least one salt and the at least one high molecular weight, non-ionic, hydrophilic polymer to an aqueous solvent and allowing sufficient time for the components to form a homogeneous mixture, with or without subjecting the composition to mechanical mixing. Alternatively, some or all of the aqueous solvent can be added to one, some, or all of the components to create a homogeneous mixture. The aqueous basic composition may be used directly (after sterilization, for example by autoclaving or filtration) or may be subjected to a drying process, such as lyophilization, to create a dried composition. The basic dried composition should be sterile, sterilization occurring either while in the liquid state or after drying (e.g., by gamma irradiation or other known techniques for sterilizing solid compositions). The basic dried composition can be reconstituted or rehydrated (these terms used interchangeably herein) for use by dissolving it in a sterile aqueous liquid, such as water or an aqueous buffer, to create a basic liquid composition according to the invention.

It will be immediately apparent to the skilled artisan that a complex composition according to the invention can be prepared in the same manner as a basic composition, the main difference being the number of substances mixed in the composition. The method of making a complex composition according to the invention does not include adding platelets or platelet microparticles to the composition.

Parameters and techniques for lyophilizing the mixture can include those that are well-known to the skilled artisan. For example, any of the lyophilization parameters and techniques disclosed in U.S. Patents Nos. 7,811,558, 8,097,403, and 8,486,619 (all of which are hereby incorporated by reference into this document) can be used. Non-limiting additional or alternative parameters and additional techniques are discussed below, or are well known in the art.

When reconstituted, the pH of the complex composition may be any pH that is suitable for stability of the composition and administration to a subject. Accordingly, it can range from mildly acidic to mildly basic. Exemplary ranges and specific pHs are those that fall within pH 5.0 to pH 9.0. In various embodiments, the pH of the complex composition is 5.0, 5.5, 6.0, 6.5, 7.0, 7.5, 8.0, or up to 9.0. In other embodiments, the pH is any pH within the range of 5.0 to 9.0, including any range taken from among the specific pH values stated directly above. In embodiments relating to complex solid compositions, the complex compositions may comprise one or more substances that, when hydrated, cause the pH of the resulting complex liquid composition to be in a suitable range.

In embodiments, after drying, such as by lyophilization, the compositions may be heated, such as at about 50°C, 55°C 60°C, 65°C, 70°C, 75°C, 80°C, 85°C, or 90°C. In embodiments, the temperature is any temperature within the range of room temperature to about 90°C. Yet further, in embodiments where the composition is in a dry state and is to be heated, the composition can be heated from less than one minute up to 24 hours or more. Accordingly, the time of heating can be, for example, 0, 2, 4, 8, 12, or 24 hours. In other embodiments, the time of heating is any time within the range of less than 1 minute to 24 hours, including any minute or fraction thereof within that range. As non-limiting examples, the post-drying heat treatment step can be at about 80°C for about 24 hours, about 80°C for about 12 hours, at about 70°C for about 24 hours, and about 70°C for about 12 hours. In preferred embodiments, the composition is sterilized using standard sterilization techniques, such as heating or irradiation. Sterilization can also be accomplished using filtration of the liquid form of the composition prior to lyophilization or other techniques that remove water to provide a solid, including a dry, formulation. Virus reduction or removal is typically accomplished by filtration.

In an aspect of the invention, the present invention provides a method for treating loss of fluids in a subject that causes reduced volume and/or pressure in the systemic circulation, the method comprising administering to the subject an effective and tolerable amount of a composition of the invention. As a practical matter, administering will be by way of infusion or injection of a liquid composition according to the invention into the blood circulation system of the subject. In embodiments, the method of treating can be considered a method for restoring the blood volume, blood pressure, or both in a subject in need to adequate levels to sustain life. In embodiments, the method alternatively or additionally restores organ blood flow to a point that sustains organ function of a target organ. In embodiments, the blood volume and/or blood pressure, and/or organ blood flow in the subject before administration of the composition are lower than levels considered normal in the population to which the subject belongs. However, in prophylaxis treatments, the composition can be delivered prior to the development of any of the disorders mentioned herein for the purpose of preserving all or some of these functions in anticipation of a likely necessity. The term fluid as used herein refers to a liquid originating inside a subject, such as interstitial and extracellular fluid, lymph, and blood. "Normal levels" of human blood pressure as used herein refer to a systolic blood pressure that is between approximately 80 to approximately 120 mm Hg and a diastolic blood pressure that is between approximately 60 to approximately 79 mm Hg. "Low levels" of human blood pressure as used herein refer to blood pressure that is lower than approximately 90 mm Hg, systolic, and approximately 60 mm Hg, diastolic. Other "normal" and "low" levels of blood pressure for various other species of animals are known to those of skill in the art, and need not be listed herein. It is also to be understood that the method can be a method of delivering drugs or other beneficial substances systemically to a subject.

In some embodiments, the subject being treated is characterized as having hypovolemia. In embodiments, the hypovolemia is caused by at least one condition selected from blood loss by hemorrhage following trauma or injury (including deliberate surgical procedures), uncontrolled blood loss, bleeding, dehydration due to heat-induced volume loss (uncompensated by fluid restoration), fluid loss associated with a burn injury, neurogenic diabetes insipidus, and uncontrolled diabetes and other medical conditions associated with impaired water regulation. Contraction of bodily fluids can also be the result of pharmacological treatments (e.g., diuretics) or genetic syndromes, which can be addressed by the invention. In still other embodiments, the subject has a disease that causes Viral Hemorrhagic Fever (VHF), such as VHF due to infection by the Ebola virus, Dengue virus, yellow fever virus, and other infectious conditions, such as hemorrhagic fever with renal syndrome (HFSRS). Furthermore, acute radiation sickness (ARS), a syndrome known for disruption of hemostasis, could also benefit from blood volume and pressure restoration by the composition.

Administration of the compositions of the invention to a subject can be performed by any suitable route of administration, including, but not limited to, intravenously, intra-abdominally (peritoneally), direct intraventricularly, intracranially, rectally, or other modes of delivery known in the art. As disclosed above, for practical purposes, the compositions to be administered are typically liquid compositions, which are administered by way of intravenous infusion or injection. It is well within the skill and knowledge of the skilled artisan to select an appropriate administration route without undue or excessive experimentation.

It is to be understood that the act of administering can be performed more than one time to achieve a treatment regimen suitable for treatment of the diseases, disorders, and physiological states discussed herein. For example, in certain situations, maintenance of a given blood volume and/or blood pressure for an extended period of time (*e.g.,* one hour, five hours, 10 hours, or more) might be desired. In the event that the composition of the invention does not provide the desired effect for the desired length of time, a second, third, etc. administration can be performed to sustain the medical objective at the desired target, such as to again achieve the increments needed in volume and/or blood pressure to the desired levels of medical benefits. Because the compositions of the invention have not shown the adverse effect seen in other colloidal resuscitative compositions, concern for adverse effects upon multiple administrations of compositions of the present invention are unlikely.

The term "effective amount", as in "a therapeutically (or prophylactically) effective amount" of a composition of the invention refers to the amount of the composition necessary to elicit a desired biological or medical response. As will be appreciated by those of skill in the art, the effective amount of a composition can vary depending on such factors as the desired biological/medical endpoint, the specific activity of the composition to be delivered, the size of the subject, and the target organ, tissue, or cell, for example. In exemplary embodiments, the term "effective amount" refers to an amount sufficient to change the blood volume and/or blood pressure and/or organ blood flow (or any other medical benefits across the gamut of potential medical indications listed) of a subject, preferably to restore the blood volume, blood pressure, and/or organ blood flow in the subject to levels close to the normal blood volume, pressure, and organ blood flow before the loss of bodily fluids. However, in certain situations, such as surgery for trauma, it might be beneficial to raise blood pressure to a level that is below "normal" during the surgery. As with selection of the administration route, it is well within the skill and knowledge of the skilled artisan to select an appropriate amount of the composition to administer without undue or excessive experimentation.

A "subject" according to the invention is a member of any vertebrate species. Accordingly, a subject can be a human subject for medical purposes, such as for the diagnosis or treatment of an existing disease, disorder, or condition, or for the prophylactic diagnosis or treatment for preventing the onset of a disease, disorder, or condition. A subject can also be an animal subject for medical or veterinary purposes, or for developmental purposes. Suitable animal subjects include mammals including, but not limited to, non-human primates, *e.g.*, monkeys, apes, gibbons, chimpanzees, orangutans, macaques and the like; bovines, *e*.*g*., cattle, oxen, and the like; ovines, *e.g.,* sheep and the like; caprines, *e.g.,* goats and the like; porcines, *e.g.,* pigs, and the like; equines, *e.g.,* horses, donkeys, zebras, and the like; felines, including wild and domestic cats; canines, including dogs and wolves; lagomorphs, including rabbits, hares, and the like; and rodents, including mice, rats, guinea pigs, and the like. An animal may be a transgenic animal or any manipulated laboratory animal, such as in-bred animals designed for disease studies. In some embodiments, the subject is a human, including, but not limited to, fetal, neonatal, infant, juvenile, and adult subjects. Further, a subject can include a patient afflicted with or suspected of being afflicted with a disease, disorder, or condition. Subjects also include experimental animal disease models (*e.g*., rats or mice used in experiments, and the like), companion animals, or game animals.

While the invention finds advantageous use in therapeutic treatment of subjects, it is also well-suited for prophylactic treatment of subjects by administering a prophylactically-effective amount of a composition of the invention to a subject in anticipation of normal bleeding associated with surgery or to mitigate a potential medical problem that occurs during surgery. As with therapeutic treatment methods, it is well within the skill and knowledge of the skilled artisan to select an appropriate amount of the composition to administer, the route of administration, and the number of times the composition is administered to achieve a desired biological/medical prophylactic effect in a subject without undue or excessive experimentation. Non-limiting exemplary amounts for use in a rat model are provided in the Examples, below, to serve as a guide, not a limitation, for the practitioner.

In yet another aspect of the invention, diagnostic methods are provided. According to this aspect of the invention, a composition is administered to a subject in an amount that is effective for diagnosis of a disease or disorder, wherein the composition includes a detectable agent. Preferably, the detectable agent is an agent that is bound (*e.g.*, covalently coupled) to a high molecular weight, non-ionic, hydrophilic polymer present in the composition. For example, in an embodiment, the high molecular weight, non-ionic, hydrophilic polymer is bound to an imaging agent. The composition can be administered to the subject in amount sufficient to detect with the appropriate detecting device. If the subject suffers from disrupted integrity of the vascular circulation, leakage of the modified high molecular weight, non-ionic, hydrophilic polymer could be visualized by external, non-invasive methods known in the art. As a non-limiting example, positron emission tomography (PET) can be used.

Advantages of the present invention include, but are not limited to, the availability of the present composition as a sterile powder (either lyophilized or not) that can be mixed with an aqueous solvent and used in any locale and situation where such a solvent (*e.g.*, sterile water) is available; the relatively low volume of sterile solvent needed, such as approximately 1 ml or less up to hundreds of ml; the long shelf life of the formulation in its dry state; and that the oncotic pressure-generating composition of the present invention can contain relatively few chemical entities, all of which are well-defined and currently commercially available.

The ability of the present composition in its dry form to have an unusually long shelf life and to be stored, maintained, and deployed in large volumes with very little powder, allows the present invention to be used in many diverse conditions where volume expansion is rapidly and efficiently needed. For example, the present invention can be used in medical situations where standard of care (SOC) volume expanding modalities might not be available, such as combat conditions, accident sites, airline carriers, marine vessels (*e.g*., submarines, aircraft carriers, Coast Guard cutters), and the like. In particular, sustained palliative care "en route" from distant combat zones where sustaining acute resuscitation management is needed until arrival at medical facilities could save many casualties. As another example, the present invention can be used for hypovolemia due to blood loss by hemorrhage and following placement of a tourniquet after trauma or injury, hypovolemia due to dehydration such as due to heat exposure, hypovolemia due to loss of fluid associated with burn injuries or diarrhea, and conditions of excessive extracellular volume/fluid (ECV/F) accumulation in need for contraction, such as ascites fluid in the abdominal cavity. The terms "hemorrhage" and "hemorrhaging" as used herein refer to blood escaping from the circulatory system into bodily cavities or to the external environment. The term "hypovolemia" as used herein refers to a state of decreased blood volume, such as a decrease in volume of blood and plasma. Hypovolemia is often characterized by salt depletion, for example as a result of treatment with a diuretic, and thus differs from dehydration, which is excessive loss of body water.

Another aspect of the invention relates to kits. In general, a kit according to the invention comprises a composition of the invention contained within a sterile, airtight and watertight vessel, such as a vial, cannister, ampoule, bag, and the like. The composition may be in either a dry state or a liquid state. For ease of storage and shelf-life, preferred embodiments relate to dry compositions. A kit according to the invention can include one or more containers containing a composition of the invention as the sole component of the kit. Alternatively, one, some, or all of the reagents and supplies for hydrating a dry composition and/or for administering a composition to a subject can be present in packaged combination with a dry composition of the invention.

### EXAMPLES

The invention will be further explained by the following Examples, which are intended to be purely exemplary of the invention, and should not be considered as limiting the invention in any way.

Hypovolemia and/or hypotension are common medical conditions, which if not treated properly can lead to high morbidity and mortality. Numerous resuscitation fluids have been introduced into medical practice to prevent or alleviate states of shock that result from hypovolemia and/or hypotension. Currently, the most common treatments for such life-threatening conditions include infusions into the systemic circulation of electrolyte-based solutions, starch-based solutions, or albumin-based solutions. More recently, research has been performed to determine if relatively small volumes of hypertonic electrolyte solutions can be used to minimize blood volume excesses and blood electrolyte dilution, which are undesirable consequences of treatment with other solutions.

In an effort to characterize a novel basic composition and a more complex embodiment of that composition for treatment of hypotension and hypovolemia, first a complex composition was formulated and infused into rats that had been subjected to a controlled arterial bleed, and the complex composition's effects on blood pressure were measured and compared to the blood pressure in rats receiving no treatment or treatment with other resuscitative compositions. It was discovered that a complex composition of the present invention was significantly better at raising blood pressure rapidly and sustaining the increase in blood pressure in this model system than no treatment or treatment with a crystalline resuscitative fluid (normal saline; 0.9% NaCl in water). It was also found that a complex composition of the invention showed a superior, persistent, sustained effect on blood pressure increase as compared to a known colloidal resuscitative composition (HAES-steril^{®}; 6% hydroxyethyl starch).

### Materials and Methods

Experiments were performed in male Sprague-Dawley rats weighing 400 +/- 20 g. Rats were housed in cages (two rats per cage) and allowed access to food and water ad libitum until the night before the day of surgery, at which time the rats were denied food, but continued to have access to water ad libitum.

### Anesthesia and Surgical Procedures

On the day of the experiment, the rats were anesthetized by Inactin (Thiobutabarbital sodium; 100 mg/kg, intra-peritoneal - i.p.) and placed on a thermoregulated surgical table that maintains body temperature at 37°C. Following tracheotomy (for ventilation purposes), polyethylene catheters (PE₅₀) were inserted into the left femoral artery for blood pressure monitoring, and into the right jugular vein for blood sampling and infusion of various solutions, including drug infusions. In addition, a polyethylene catheter (PE₅₀) was inserted into the right carotid artery to allow for controlled bleeding of the rats.

### Experimental Methods

### Surgery

The experimental methods relied on a hypotensive/hypovolemia model in which controlled hypotension and hypovolemia were induced via blood withdrawal from the carotid artery. Briefly, following anesthesia and cannulation, the rats were subjected to controlled bleeding via the carotid artery for over five minutes, during which 6.0 - 7.0 ml of blood was drawn from each rat to achieve a target mean arterial pressure (MAP) of 45 mm Hg. The nadir of blood pressure at the end of the controlled bleeding period is considered the shock level.

### Statistical Analysis

Statistical analyses were performed by ANOVA with post-hoc analysis as appropriate on a case-by-case basis. Student t-test analysis was used for particular data points. Statistical significance was accepted at p<0.05. The number of rats in each study is indicated below and in the figures as "n=8", "n=9", etc.

### Example 1: Beneficial and Superior Effect of a Complex Composition of the Invention on Raising Blood Pressure

To initially test the effect of a complex composition of the invention on induced hypovolemia and hypotension, procedures were performed as described above in experiments comprising three protocols. The experiments included three groups of rats, as follows:

First Group: In this protocol, rats (n=8) were prepared and subjected to bleeding as described above. Blood pressure was measured and recorded immediately before the start of the bleeding period (t = -5), at the end of the bleeding period (t = 0), and periodically for two hours after the treatment period (*i.e.,* for 140 minutes after the end of the bleeding period), as described below with respect to the Second Group and the Third Group.

Second Group: In this protocol, rats (n=8) were prepared and subjected to bleeding as described above. Blood pressure was measured and recorded immediately before the start of the bleeding period (t = -5) and at the end of the bleeding period (t = 0). Ten minutes after the end of the bleeding period, the rats were infused with 6.0 - 7.0 ml (corresponding to an equal volume as the blood drawn from each individual animal) of normal saline over a ten minute period. Blood pressure was measured and recorded at five minutes into the treatment period, at the end of the treatment period, and periodically for two hours after the end of the treatment period.

Third Group: In this protocol, rats (n=8) were prepared and subjected to bleeding as described above. Blood pressure was measured and recorded immediately before the start of the bleeding period (t = -5) and at the end of the bleeding period (t = 0). Ten minutes after completion of bleeding, the rats were infused with 6.0 - 7.0 ml (corresponding to an equal volume as the blood drawn from each individual animal) of a complex aqueous composition comprising 7.1 mM HEPES buffer, 56.25 mM NaCl, 3.6 mM KCl, 9 mM NaHCO₃, 75 mM trehalose, 3.75 mM dextrose, and Ficoll^{®} 400 (6% w/v) over a ten minute period. Blood pressure was measured and recorded at the end of the treatment period, and periodically for two hours after the end of the treatment period.

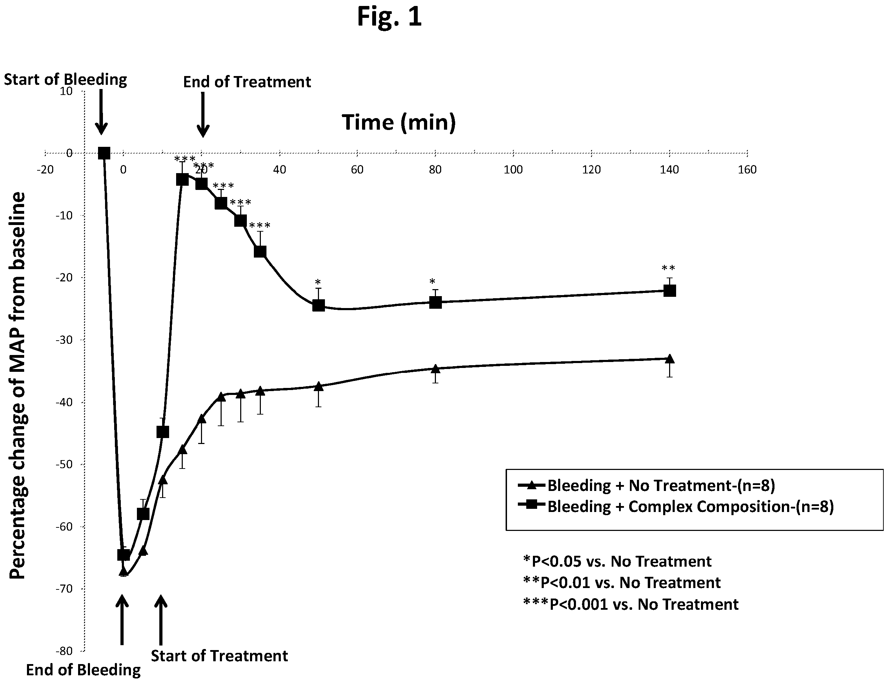

Figure 1 illustrates the percent change of mean arterial pressure (MAP) from baseline of anesthetized rats exposed to bleeding to the extent of reducing MAP to shock levels (<45 mm Hg. This figure shows the effect of infusion of a complex composition according to the invention (Third Group; represented by the line following the points denoted by squares in Figure 1) as compared to no treatment (First Group; represented by the line following the points denoted by triangles in Figure 1). The end of the five minute bleeding period is denoted by the arrow positioned at the left lower part of the figure, at t = 0. The treatment period is denoted by the arrows positioned at the left lower part of the figure (beginning of treatment; t = 10) and at the top of the figure (end of treatment; t = 20).

As can be seen from Figure 1, treatment with a complex composition of the invention rapidly increased MAP when administered over a ten minute period, starting ten minutes after termination of the controlled bleeding. The effect of the complex composition on MAP was statistically significantly superior to the innate compensatory response of untreated rats. This statistically significant difference lasted throughout the time course monitored. As such, the data support a conclusion that treatment for hypovolemia and/or hypotension with a complex composition of the present invention is an effective treatment method, and, as such, the complex composition of the present invention is an effective pharmaceutical composition for treatment of hypovolemia, hypotension, or both.

A second experiment was performed to determine the effect of a complex composition of the invention (*i.e.,* Third Group) as compared to the effect of treatment with the same volume of normal saline (Second Group) on raising blood pressure in this rat model. Normal saline is currently used in hospital and clinical settings to treat hypovolemia and hypotension. A no treatment control (First Group) was included in the experiment. The experiment was performed as described above in the first experiment, with the exception of inclusion of a saline test for comparison.

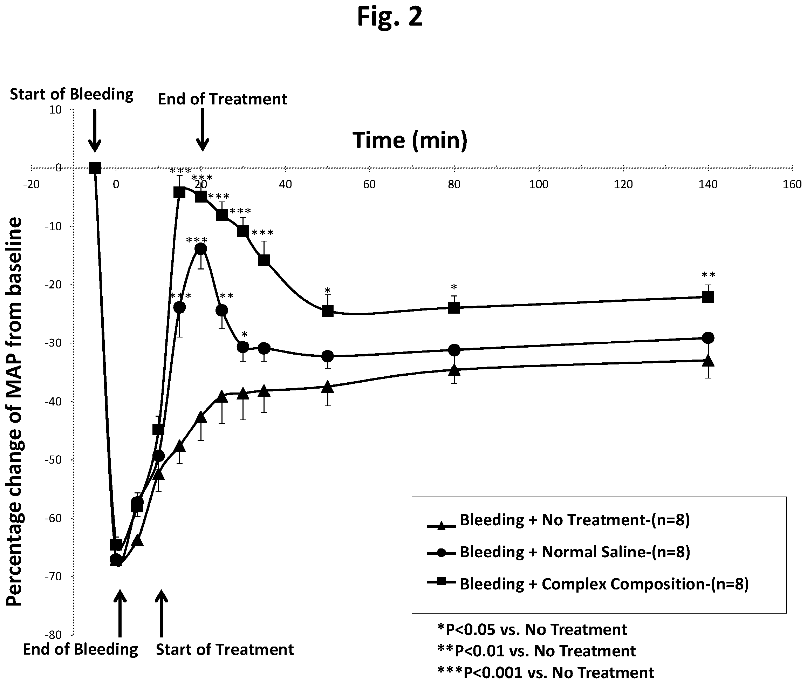

Figure 2 shows the effect of infusion of a complex composition according to the invention (Third Group; represented by the line following the points denoted by squares in Figure 2) as compared to an equal volume of normal saline (Second Group; represented by the line following the points denoted by circles in Figure 2) and no treatment (First Group; represented by the line following the points denoted by triangles in Figure 2). The end of the five minute bleeding period is denoted by the arrow positioned at the left lower part of the figure, at t = 0. The treatment period is denoted by the arrows positioned at the left lower part of the figure (beginning of treatment; t = 10) and at the top of the figure (end of treatment; t = 20).

As can be seen from Figure 2, treatment with a complex composition of the invention rapidly increased MAP when administered over a ten minute period, starting ten minutes after termination of the controlled bleeding. The effect of the complex composition on MAP was superior to the effect of an equal volume of normal saline in respect to both the extent of restoration of blood pressure and the durability of the effect. As such, the data support a conclusion that treatment for hypovolemia and/or hypotension with a complex composition of the present invention is more effective than treatment with normal saline. The complex composition of the present invention thus can be considered an effective pharmaceutical composition for treatment of hypovolemia, hypotension, or both.

### Example 2: Effect of a Complex Composition of the Invention When Used At Low Volumes

Having shown in Example 1 that a complex composition of the invention is effective at restoring MAP rapidly and to a greater extent and for a longer time than no treatment or treatment with normal saline when both are administered at an equal volume of the volume of blood removed from the rats, the effect of increasingly lower volumes of a complex composition of the invention was tested. Three additional Groups of rats were used for this experiment, as follows:

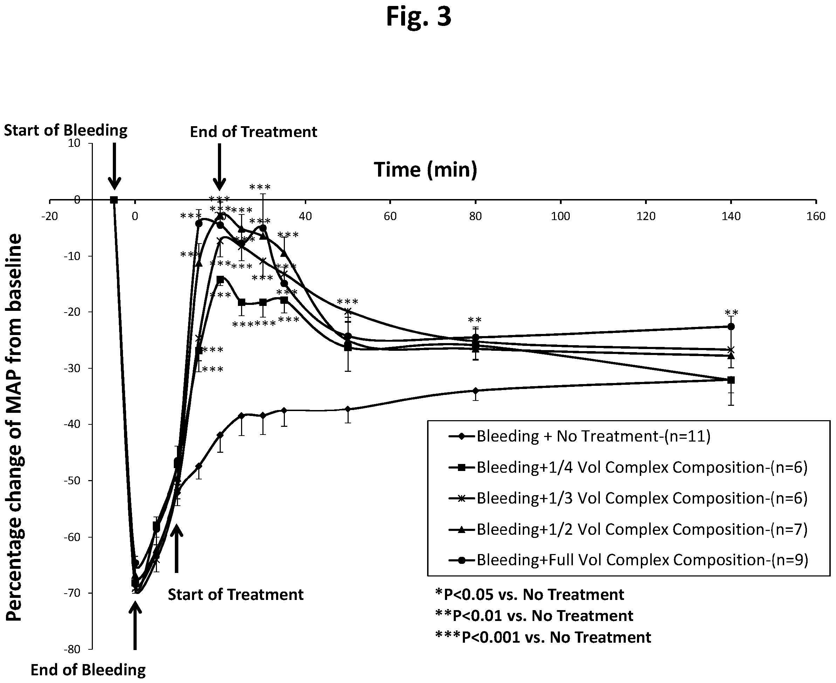

Fourth Group: In this protocol, rats (n=7) were prepared and subjected to bleeding as described above, with removal of approximately 6.0 - 7.0 ml of blood over a five minute period. Blood pressure was measured and recorded immediately before the start of the bleeding period (t = -5) and at the end of the bleeding period (t = 0). Ten minutes after completion of bleeding, the rats were infused with 3.5 ml of a complex aqueous composition comprising 7.1 mM HEPES buffer, 56.25 mM NaCl, 3.6 mM KCl, 9 mM NaHCO₃, 75 mM trehalose, 3.75 mM dextrose, and Ficoll^{®} 400 (6% w/v) over a ten minute period. Blood pressure was measured and recorded at the start of the treatment period (t = 10), at the end of the treatment period (t = 20), and periodically for two hours after the end of the treatment period. This Group is referred to in accompanying Figure 3 as "Bleeding + 1/2 Vol Complex Composition".

Fifth Group: In this protocol, rats (n=6) were prepared and subjected to bleeding as described above, with removal of approximately 6.0 - 7.0 m of blood over a five minute period. Blood pressure was measured and recorded immediately before the start of the bleeding period (t = -5) and at the end of the bleeding period (t = 0). Ten minutes after completion of bleeding, the rats were infused with 2.3 ml of a complex aqueous composition comprising 7.1 mM HEPES buffer, 56.25 mM NaCl, 3.6 mM KCl, 9 mM NaHCO₃, 75 mM trehalose, 3.75 mM dextrose, and Ficoll^{®} 400 (6% w/v) over a ten minute period. Blood pressure was measured and recorded at the start of the treatment period (t = 10), at the end of the treatment period (t = 20), and periodically for two hours after the end of the treatment period. This Group is referred to in accompanying Figure 3 as "Bleeding + 1/3 Vol Complex Composition".

Sixth Group: In this protocol, rats (n=6) were prepared and subjected to bleeding as described above, with removal of approximately 6.0 - 7.0 ml of blood over a five minute period. Blood pressure was measured and recorded immediately before the start of the bleeding period (t = -5) and at the end of the bleeding period (t = 0). Ten minutes after completion of bleeding, the rats were infused with 1.75 ml of a complex aqueous composition comprising 7.1 mM HEPES buffer, 56.25 mM NaCl, 3.6 mM KCl, 9 mM NaHCO₃, 75 mM trehalose, 3.75 mM dextrose, and Ficoll^{®} 400 (6% w/v) over a ten minute period. Blood pressure was measured and recorded at the start of the treatment period (t = 10), at the end of the treatment period (t = 20), and periodically for two hours after the end of the treatment period. This Group is referred to in accompanying Figure 3 as "Bleeding + 1/4 Vol Complex Composition".

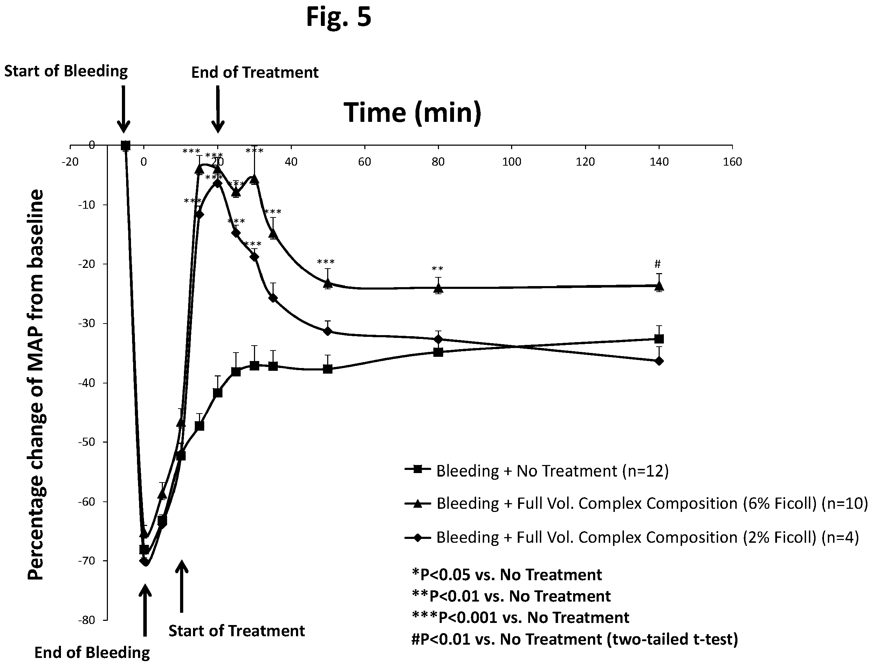

Figure 3 shows line graphs indicating the percent change of MAP from baseline over a time course of 140 minutes for each of the First Group and Third through Sixth Groups. Consistent with Figures 1, 3, and 5 (Figure 5 is discussed below), restoration of MAP to about 30% below baseline was slowly achieved over 140 minutes in rats receiving no treatment (First Group; indicated by the line drawn between points represented by diamonds in Figure 3). Also consistent with Figures 1, 3, and 5, treatment of rats with a volume of a complex composition of the invention equal to the volume drawn from the rats (Third Group; indicated by the line drawn between points represented by circles in Figure 3) provided a rapid and long-lasting statistically significant restoration of MAP as compared to no treatment. Interestingly, treatment of rats with a volume of a complex composition of the invention representing one-half (Fourth Group; indicated by the line drawn between points represented by triangles in Figure 3), and one-third (Fifth Group; indicated by the line drawn between points represented by squares in Figure 3) of the volume of blood drawn from the rats provided a rapid and robust restoration of MAP as compared to no treatment. The restoration of MAP in the group that received a composition of the invention representing one-fourth of the volume of blood drawn from the rats (Sixth Group; indicated by the line drawn between points represented by Xs in Figure 3) was similarly rapid as seen in rats treated with a full volume of the composition, one-half volume of the composition, and one-third volume of the composition, although restoration was not as great as seen in the other groups.

The data presented in Figure 3 support certain conclusions. First, restoration of MAP as a result of administration of a complex composition of the present invention is rapid and robust, providing a statistically significant improvement over no treatment at least out to 140 minutes for a full-volume replacement, and at least out to 50 minutes for replacement volumes of one-half, one-third, and one-fourth of the volume of blood drawn from the rats. Next, restoration of MAP was achieved using a complex composition of the invention at a volume equal to the amount drawn from the rats, at one-half the volume drawn from the rats, at one-third the volume drawn from the rats and at one-fourth of the volume drawn from the rats. These data support the conclusion that it is the components of the complex composition, not volume replacement alone, that is causing restoration of MAP above and beyond volume replacement as represented by the normal saline volume replacement. And further, a comparison of Figure 3 and Figure 2 shows that restoration of MAP using a complex composition of the invention that is one-fourth of the volume drawn from the rats provides a similar immediate restoration of MAP as provided by a full volume replacement with normal saline. However, the duration of the effect is longer with the complex composition of the invention, even at one-fourth of volume replacement. Yet further, the data relating to administration of one-half, one-third, and one-fourth volumes of a complex composition of the invention, coupled with the canine safety data presented in Table 1, above, support the conclusion that multiple administrations can be performed, if desired, without serious concern for effects due to introduction of excessive liquid into a subject or adverse kidney effects. For example, if the practitioner elected to administer one-fourth of the volume of blood lost by a subject, at a later time (*e.g.,* 60 minutes later), another one-fourth volume could be administered. As such at the later time, MAP would remain high due to the second administration, yet the subject would still have received only one-half of the volume of blood lost. Such a feature is extremely advantageous in situations where immediate surgical intervention is not possible.

### Example 3: Comparison of a Complex Composition of the Invention with Commercially Available Colloidal Solution for Treatment of Hypovolemia

The data presented in Figures 1 - 3 and discussed above show that a complex composition of the invention is effective at rapidly restoring MAP and maintaining that restoration over a clinically meaningful period of time. The data also show that a complex composition of the invention is superior to normal saline at restoring MAP at composition volumes as low as one-third of the volume drawn from the rats (when compared to administration of a full volume of normal saline). And further, a complex composition administered at one-fourth the volume drawn from the rats has a similar immediate effect, but a longer lasting robust effect, as administration of a full volume of saline. To determine the relative effects of a complex composition of the present invention and a commercially available hydroxyethyl starch, sold under the brand name HAES-steril^{®}, the protocol relating to the Third Group above was used, and one additional Group of rats was used for this experiment, as follows:

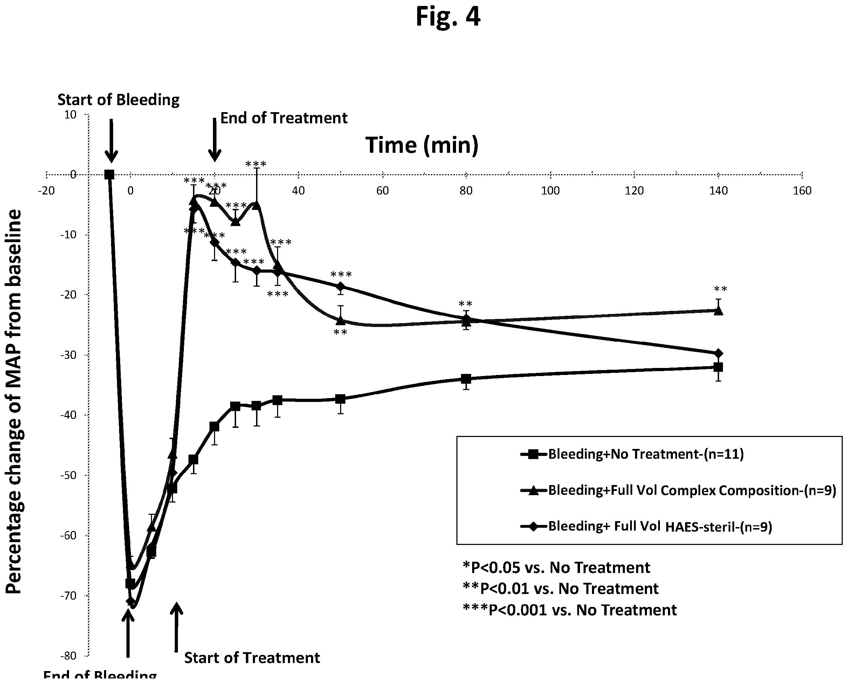

Seventh Group: In this protocol, rats (n=9) were prepared and subjected to bleeding as described above, with removal of approximately 6.0 - 7.0 ml of blood over a five minute period. Blood pressure was measured and recorded immediately before the start of the bleeding period (t = -5) and at the end of the bleeding period (t = 0). Ten minutes after completion of bleeding, the rats were infused with 6.0 - 7.0 ml of the commercially-available HAES-steril^{®} product over a ten minute period. Blood pressure was measured and recorded at the start of the treatment period (t = 10), at the end of the treatment period (t = 20), and periodically for two hours after the end of the treatment period. This Group is referred to in accompanying Figure 4 as "Bleeding + Full Vol HAES-steril".

As can be seen in Figure 4, a complex composition of the present invention, when administered at the same volume as the volume drawn from the rats (*i.e.,* Third Group; indicated by the line drawn between points represented by triangles), showed a rapid increase in MAP as compared to baseline, which was sustained at a statistically significant greater level than the level seen in rats receiving no treatment. Similarly, HAES-steril^{®}, when administered at the same volume as the volume drawn from the rats (*i.e.,* Seventh Group; indicated by the line drawn between points represented by diamonds), showed a rapid increase in MAP as compared to baseline, which was sustained for about 30 minutes at a statistically significant greater level than the level seen in rats receiving no treatment. Figure 4 thus shows that a composition of the present invention is at least as effective at raising MAP than a commercially available product.

### Example 4: Determination of Effect on MAP of Complex Compositions of the Invention Having Different Concentrations of High Molecular Weight, Non-Ionic, Hydrophilic Polymer

The data discussed above and presented in Figures 1-4 fully support the conclusions that complex compositions according to the present invention are not only effective in rapidly restoring MAP to hypovolemic subjects, but that in certain volumes (as compared to volumes of blood loss by the subjects), provide a robust and long-lasting improvement in MAP. Indeed, surprisingly small volumes of the complex composition of the invention provide significant benefits, at least over the acute phase of hypovolemia.

In this Example, the effect of the concentration of a high molecular weight, non-ionic, hydrophilic polymer in a complex composition of the invention was investigated. That is, rather than varying the volume of complex composition administered to a subject as disclosed above, in this Example, full volumes (*i.e.,* volumes of complex compositions that are equal or nearly equal to volumes of blood lost by the animal) were administered after blood removal in the rat model disclosed above. Specifically, two complex compositions according to the invention were prepared. The first complex composition was the composition described as being used in the Third Group of Example 1, above. The second complex composition was identical to the composition used in the Third Group of Example 1, above, with the exception that the concentration of the high molecular weight, non-ionic, hydrophilic polymer was reduced from 6% (w/v) to 2% (w/v). For ease of reference, these two compositions are referred to herein as "Complex Composition (6% Ficoll)" and "Complex Composition (2% Ficoll)", respectively.

A study to determine the effect of the two complex compositions was performed as disclosed above using the rat model used for all previous studies reported herein. The results of the study is depicted in Figure 5. As can be seen in Figure 5, treatment of controlled bleeding-induced hypovolemic rats with both Complex Composition (6% Ficoll) and Complex Composition (2% Ficoll) provides a statistically insignificant difference in recovery of MAP during the treatment phase (*i.e.,* the first ten minutes following the start of infusion of the compositions), a recovery that is statistically significantly superior to no treatment and to treatment with normal saline (compare Figure 5 to Figure 2). The superior effect of Complex Composition (2% Ficoll) appears to be, however, short-lived, and the significant improvement is substantially lost by 50 minutes post treatment. However, although the significantly superior effect of Complex Composition (2% Ficoll) does not appear to be a long-term effect, the data support a conclusion that, even at 2% (w/v) of a high molecular weight, non-ionic, hydrophilic polymer, a relatively short-term, acute phase beneficial effect on restoration of MAP that is statistically significant as compared to no treatment is provided by a complex composition of the present invention. Indeed, that beneficial effect could be the difference between life and death of a subject suffering from hypovolemia or hypotension.

The data presented in Figure 5, coupled with the data presented in the other figures and in Table 1, strongly support the conclusion that complex compositions of the present invention provide superior resuscitation of blood volume and pressure as compared to certain resuscitation compositions known in the art. The present complex compositions further are shown to provide superior or comparable resuscitation of blood volume and pressure as compared to other colloid compositions, without the adverse side effects of those other colloid compositions.

### Example 5: Performance of a Basic Composition of the Invention

The previous Examples and corresponding Figures show that at least two embodiments of a basic composition (*i.e.,* at least two complex compositions, one including a high molecular weight, non-ionic, hydrophilic polymer at 6% (w/v) and another including a high molecular weight, non-ionic, hydrophilic polymer at 2% (w/v)) according to the present invention provide surprising and unexpected medically and clinically important improvements in the treatment of hypovolemia and hypotension in a rat model that is a commonly used model for human hypovolemia and hypotension research. To confirm that the results obtained using the complex compositions are representative of the results that can be obtained using the basic composition of the invention, experiments were performed to compare the effects of a basic compositions of the invention at two different volumes to two similar complex compositions of the invention, which were proven above to be suitable, and even superior, for use in treating hypovolemia and/or hypotension.

More specifically, for this Example, a basic composition of an embodiment of the invention was produced. The basic composition of this embodiment comprised normal saline containing 6% (w/v) Ficoll400, which was prepared as a liquid composition, lyophilized, and sterilized with heat, as described above. For the experiments described below, the basic composition was administered to a rat using the rat model described above and using either i) an equivalent volume of basic composition as compared to the amount of blood bled from the rat, or ii) administered to the rat at a volume of one-fourth of the volume of the blood bled from the rat, as will be made more clear below. For comparison, an equivalent volume of the complex composition of the first complex composition described as being used in the Third Group of Example 1, above, was used in a volume-to-volume manner as per the basic composition.

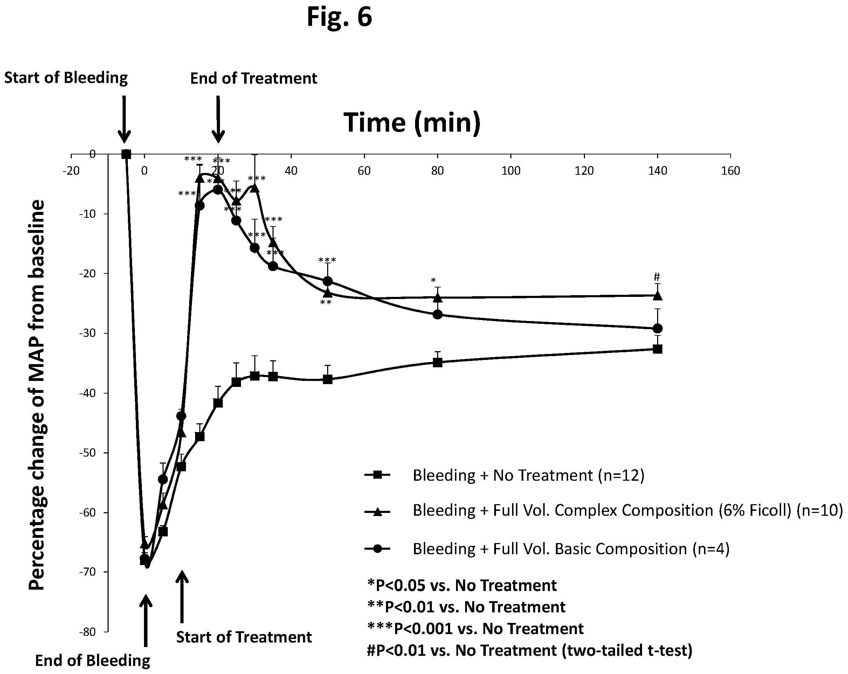

In the first experiment, a full volume of the exemplary basic composition was infused into rats in accordance with the disclosure above relating to infusion of a full volume of a composition as compared to the volume of blood removed from the animal. Blood pressure was recorded at the start of bleeding (t = -5) and at the end of bleeding (t = 0). Treatment with a volume of the complex composition and with the basic composition began independently at 10 minutes after cessation of bleeding (t = 10), and blood pressure was recorded as in previous studies reported herein. Treatment (administration) for both compositions was performed by blood stream infusion for 10 minutes. At ten minutes after starting treatment, MAP was monitored as described above. The results are presented in Figure 6.

As can be seen in Figure 6, treatment with a basic composition provides essentially the same beneficial effect in raising MAP during the first 50 minutes as does treatment with a complex composition. Although the statistically significant improvement does not appear to extend much beyond 50 minutes, the improvement can still be important in saving lives during transport of trauma victims from the site of trauma to a hospital.

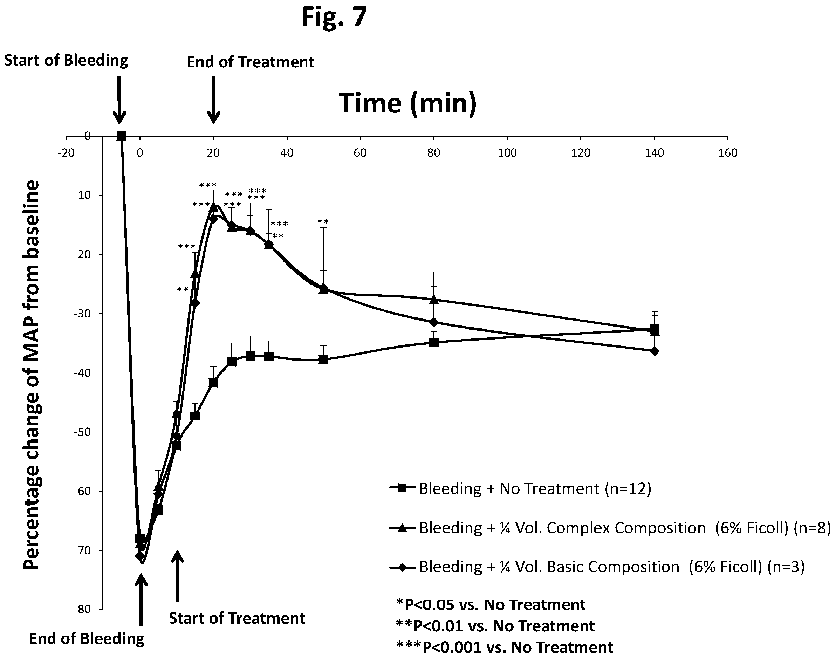

In a second experiment to determine the effect of a basic composition on resuscitation, the exemplary basic composition was infused into rats in accordance with the disclosure above. In this experiment, the basic composition was infused at one-fourth of the volume of the volume of blood lost during the bleeding phase. At the same time, the exemplary complex composition described above was infused into other rats at one-fourth of the volume of blood lost during the bleeding phase. MAP was monitored for both sets of animals as described above. The results are presented in Figure 7.

As can be seen in Figure 7, a robust initial restoration of MAP was seen for both the basic composition and the complex composition upon infusion at one-fourth the volume of blood loss. However, consistent with the data presented above in Figure 3 regarding a complex composition, the beneficial effects of infusion with this low volume replacement was diminished at about 50 minutes post treatment.

The data presented in Figures 6 and 7 support the conclusions that resuscitation of MAP after induction of hypovolemic - hypotension by treatment with a basic composition of the invention (Ficoll400 (6% w/v) dissolved in 0.9% (normal) saline) either by full volume resuscitation or one-fourth volume resuscitation resulted in virtually the same responses as noted with respective volumes of a complex composition of the invention. This includes the extent of acute MAP recovery (first 30-40 min), the kinetics of the response, and its durability over the 140 min duration of the experiment. A basic composition maintains the full activity as compared to a complex composition with respect to MAP resuscitation in this rat model of hypovolemic hypotension. The similarities are manifested in the acute phase (first 30-40 min after onset of the treatments) and along the duration of the experiment. The similarities are also preserved when fractional treatment (one-fourth) of the resuscitation volume is administered. These data suggest that in regard to MAP resuscitation, substances present in a complex composition beyond the salt and the high molecular weight, non-ionic, hydrophilic polymer, while possibly providing some advantageous properties, play little role in MAP response to treatment.

It will be apparent to those skilled in the art that various modifications and variations can be made in the practice of the present invention without departing from the scope or spirit of the invention. Other embodiments of the invention will be apparent to those skilled in the art from consideration of the specification and practice of the invention. For example, the skilled artisan will recognize that liquid compositions having higher concentrations, but the same or a similar ratio, of the components disclosed and exemplified herein can be produced, and that these liquid compositions can be administered to a subject at even lower volumes (relative to the

## Claims

1. A liquid composition comprising:
7.1 mM HEPES buffer,
56.25 mM NaCl,
3.6 mM KCl,
9 mM NaHCO₃,
75 mM trehalose,
3.75 mM dextrose, and
2% w/v polysucrose 400.

2. A solid composition prepared from the liquid composition of claim 1, optionally wherein the solid composition is a freeze-dried powder.

3. A kit comprising the composition of claim 2 contained within a sterile, airtight and watertight vessel.

4. A kit comprising the composition of claim 1 contained within a sterile, airtight and watertight vessel.

5. A composition for use in a method for treating loss of fluids in a subject in need thereof, wherein the loss of fluids causes reduced volume and/or pressure in the subject's systemic circulation, the method comprising administering to the subject the composition of claim 1.

6. The composition for use of claim 5, wherein administering the composition to the subject partially or completely restores blood flow to at least one organ in the subject.

7. The composition for use of claim 5, wherein the step of administering comprises intravenous infusion or injection of the liquid composition into the subject.

8. The composition for use of claim 5, wherein the subject is suffering from a disease or disorder that does not have a clinical symptom relating to blood volume or blood pressure.

9. The composition for use of claim 5, wherein the subject has at least one of the following conditions: hypovolemia, excessive sodium ions, and excessive extracellular volume/fluid (ECV/F), optionally wherein the hypovolemia is caused by at least one of the following conditions: blood loss by hemorrhage following trauma, injury, or a medical procedure or intervention; dehydration due to heat, and fluid loss associated with a burn injury.

10. The composition for use of claim 5, wherein the subject has a disease caused by an infectious agent, optionally wherein the infectious agent causes Viral Hemorrhagic Fever (VHF), further optionally wherein the VHF is caused by an Ebola virus.

## Patentansprüche

1. Flüssige Zusammensetzung, umfassend: 7,1 mM HEPES-Puffer,
56,25 mM NaCl,
3,6 mM KCl,
9 mM NaHCO₃,
75 mM Trehalose,
3,75 mM Dextrose und
2 % Gew./Vol. Polysucrose 400.

2. Feste Zusammensetzung, die aus der flüssigen Zusammensetzung gemäß Anspruch 1 hergestellt ist, wobei die feste Zusammensetzung gegebenenfalls ein gefriergetrocketes Pulver ist.

3. Kit, umfassend die Zusammensetzung nach Anspruch 2, die in einem sterilen, luftdichten und wasserdichten Gefäß enthalten ist.

4. Kit, umfassend die Zusammensetzung nach Anspruch 1, die in einem sterilen, luftdichten und wasserdichten Gefäß enthalten ist.

5. Zusammensetzung zur Verwendung in einem Verfahren zur Behandlung von Flüssigkeitsverlust bei einem Subjekt, das dessen bedarf, wobei der Flüssigkeitsverlust reduziertes Volumen und/oder reduzierten Druck im Körperkreislauf des Subjekts bewirkt, wobei das Verfahren das Verabreichen der Zusammensetzung gemäß Anspruch 1 an das Subjekt umfasst.

6. Zusammensetzung zur Verwendung nach Anspruch 5, wobei Verabreichen der Zusammensetzung an das Subjekt den Blutfluss zu mindestens einem Organ in dem Subjekt teilweise oder vollständig wieder herstellt.

7. Zusammensetzung zur Verwendung nach Anspruch 5, wobei der Schritt des Verabreichens intravenöse Infusion oder Injektion der flüssigen Zusammensetzung in das Subjekt umfasst.

8. Zusammensetzung zur Verwendung nach Anspruch 5, wobei das Subjekt an einer Erkrankung oder Störung leidet, die kein klinisches Symptom im Zusammenhang mit Blutvolumen oder Blutdruck aufweist.

9. Zusammensetzung zur Verwendung nach Anspruch 5, wobei das Subjekt mindestens einen der folgenden Zustände aufweist: Hypovolämie, Überschuss an Natriumionen und Überschuss an extrazellulärem Volumen/extrazellulärer Flüssigkeit (ECV/F), wobei gegebenenfalls die Hypovolämie durch mindestens einen der folgenden Zustände bewirkt wird: Blutverlust durch Hämorrhagie nach Trauma, Verletzung oder medizinischem Eingriff oder medizinischer Intervention; Dehydratisierung infolge von Wärme und Flüssigkeitsverlust im Zusammenhang mit einer Brandverletzung.

10. Zusammensetzung zur Verwendung nach Anspruch 5, wobei das Subjekt eine durch ein infektiöses Agens bewirkte Erkrankung aufweist, wobei das infektiöse Agens gegebenenfalls virales hämorrhagisches Fieber (VHF) bewirkt, wobei das VHF des Weiteren gegebenenfalls durch ein Ebola-Virus bewirkt wird.

## Revendications

1. Composition liquide comprenant :
du tampon HEPES 7,1 mM,
du NaCl 56,25 mM,
du KCl 3,6 mM,
du NaHCO₃ 9 mM,
du tréhalose 75 mM,
du dextrose 3,75 mM, et
2 % p/v de polysaccharose 400.

2. Composition solide préparée à partir de la composition liquide selon la revendication 1, éventuellement la composition solide étant une poudre lyophilisée.

3. Kit comprenant la composition selon la revendication 2 contenue à l'intérieur d'un récipient stérile, étanche à l'air et étanche à l'eau.

4. Kit comprenant la composition selon la revendication 1 contenue à l'intérieur d'un récipient stérile, étanche à l'air et étanche à l'eau.

5. Composition pour une utilisation dans un procédé pour le traitement de la perte de fluides chez un sujet qui en a besoin, la perte de fluides causant un volume et/ou une pression réduit(e)(s) dans la circulation systémique du sujet, le procédé comprenant l'administration au sujet de la composition selon la revendication 1.

6. Composition pour une utilisation selon la revendication 5, l'administration de la composition au sujet rétablissant partiellement ou complètement la circulation sanguine vers au moins un organe chez le sujet.

7. Composition pour une utilisation selon la revendication 5, l'étape d'administration comprenant une perfusion ou injection intraveineuse de la composition liquide dans le sujet.

8. Composition pour une utilisation selon la revendication 5, le sujet souffrant d'une maladie ou d'un trouble qui n'a pas de symptôme clinique relatif/relative au volume de sang ou à la pression sanguine.

9. Composition pour une utilisation selon la revendication 5, le sujet ayant au moins l'une des affections suivantes : une hypovolémie, des ions sodium en excès, et un volume extracellulaire/fluide (ECV/F) excessif, éventuellement, l'hypovolémie étant causée par au moins l'une des affections suivantes : perte de sang par hémorragie à la suite d'un traumatisme, d'une blessure, ou d'une opération ou intervention médicale ; déshydratation due à la chaleur, et perte de fluide associée à une brûlure.

10. Composition pour une utilisation selon la revendication 5, le sujet ayant une maladie causée par un agent infectieux, éventuellement l'agent infectieux causant une fièvre hémorragique virale (VHF), éventuellement en outre la VHF étant causée par un virus Ebola.
